(19) Europäisches Patentamt
European Patent Office
Office européen des brevets

(11) EP 1 098 931 B1

(12) **FASCICULE DE BREVET EUROPEEN**

(45) Date de publication et mention de la délivrance du brevet:
**09.10.2002 Bulletin 2002/41**

(21) Numéro de dépôt: **99930966.9**

(22) Date de dépôt: **16.07.1999**

(51) Int Cl.[7]: **C08L 5/08**, C08L 5/00, A23L 1/302, A23L 1/303, A61K 9/06, A61K 31/07, A61K 47/36

(86) Numéro de dépôt international:
**PCT/CA99/00651**

(87) Numéro de publication internationale:
**WO 00/004086 (27.01.2000 Gazette 2000/04)**

(54) **HYDROGELS POLYIONIQUES A BASE DE XANTHANE ET CHITOSANE POUR STABILISATION ET RELARGAGE CONTROLE DES VITAMINES**

POLYIONISCHE HYDROGELE AUF DER BASIS VON CHITOSAN UND XANTHANGUMMI ZUR STABILISIERUNG UND KONTROLLIERTER FREISETZUNG VON VITAMINEN

POLYIONIC HYDROGELS BASED ON XANTHAN AND CHITOSAN FOR STABILISING AND CONTROLLED RELEASE OF VITAMINS

(84) Etats contractants désignés:
**AT BE CH CY DE DK ES FI FR GB GR IE IT LI LU MC NL PT SE**
Etats d’extension désignés:
**AL LT LV MK RO SI**

(30) Priorité: **17.07.1998 CA 2243619**

(43) Date de publication de la demande:
**16.05.2001 Bulletin 2001/20**

(73) Titulaire: **Kemestrie Inc.**
**Sherbrooke, Québec J1L 2C8 (CA)**

(72) Inventeurs:
- **CHORNET, Esteban**
**Sherbrooke, Québec J1L IH3 (CA)**
- **DUMITRIU, Severian**
**Sherbrooke, Québec J1L 1V7 (CA)**

(74) Mandataire: **Ballot, Paul**
**Cabinet Ballot**
**122, rue Edouard Vaillant**
**92593 Levallois-Perret Cedex (FR)**

(56) Documents cités:
**US-A- 3 833 744** **US-A- 5 620 706**

- **CHEMICAL ABSTRACTS, vol. 120, no. 21, 23 May 1994 (1994-05-23) Columbus, Ohio, US; abstract no. 268799, "Preparation of soybean protein gel" XP002116026 & JP 06 022701 A (FUJI OIL CO LTD) 1 February 1994 (1994-02-01)**

Il est rappelé que: Dans un délai de neuf mois à compter de la date de publication de la mention de la délivrance du brevet européen, toute personne peut faire opposition au brevet européen délivré, auprès de l’Office européen des brevets. L’opposition doit être formée par écrit et motivée. Elle n’est réputée formée qu’après paiement de la taxe d’opposition. (Art. 99(1) Convention sur le brevet européen).

Printed by Jouve, 75001 PARIS (FR)

EP 1 098 931 B1

## Description

La présente invention a trait au domaine des hydrogels à base de xanthane et chitosane. Plus précisément, l'invention a trait aux domaines de l'alimentation et de la dermatologie où de tels hydrogels sont chargés de principes actifs tels vitamines, acides nucléiques, acides aminés, tripeptides.

Il est connu de fabriquer des hydrogels à partir de chitosane et de xanthane. Les brevets américains 5,620,706 et 5,648252 présentent de tels hydrogels utilisés comme support inerte pour l'immobilisation d'enzymes ou pour la libération contrôlée de certains antibiotiques ou agents anti-cancer. Cependant, l'application d'hydrogels de chitosane et de xanthane pour chargement, stabilisation et relargage de vitamines, acides nucléiques, acides aminés et tripeptides est jusqu'à présent inexplorée.

Une des problématiques courantes dans la fabrication de compléments alimentaires ainsi qu'en dermatologie est la conservation d'ingrédients actifs très sensibles à la dégradation tels que vitamines, acides nucléiques, acides aminés, et tripeptides, L'exposition à la lumière et la chaleur accélère cette dégradation.

Compte tenu de l'important potentiel bénéfique de ces ingrédients actifs, plusieurs véhicules tels comprimés, capsules, gellules, crèmes, onguents, gels, dispersions aqueuses (émulsions) et solutions, furent développées dans le but d'offrir une protection des produits actifs contre la dégradation ou pour les rendre hydrophobes. Cependant, plusieurs désavantages de ces préparations synthétiques, tels le potentiel irritant et la toxicité furent soulignés.

Faute de trouver un véhicule adéquat parmi les excipients conventionnels, l'utilisation de polycations synthétiques fut récemment proposée. Par exemple, la demande de brevet européen 504 066 A1, 1992, déposée par L'Oréal, France, propose des compositions cosmétiques contenant une dispersion de particules solides actives dont la surface est revêtue d'un polymère cationique. Cependant, le but du revêtement cationique est de rendre la composition stable dans son ensemble par opposition à stabiliser un ingrédient faisant face à une tendance à la dégradation.

Un important besoin demeure donc pour la découverte d'un nouveau véhicule utile en préparations alimentaires, cosmétologie et dermatologie pour contenir des ingrédients actifs photosensibles et thermosensibles tels vitamines, acides nucléiques, acides aminés et tripeptides. Un des buts de la présente invention est donc d'enseigner des préparations alimentaires et dermatologiques contenant un hydrogel polyionique naturel permettant la protection d'ingrédients actifs photo et thermo sensibles sans toutefois démontrer un potentiel irritant ou toxique. Un autre but connexe est l'enseignement d'une méthode permettant l'introduction de ces ingrédients actifs dans un hydrogel polyionique naturel.

D'autres buts et objets de la présente invention deviendront apparents à la lecture de la description détaillée qui suit. Il importe de noter que cette description détaillée présente des incorporations et exemples préférentiels de l'invention et est fournie à titre d'illustration. Ceux spécialisés dans le domaine de l'invention pourront apporter plusieurs aménagements et modifications à ces incorporations et exemples préférentiels sans toutefois dépasser le cadre de la présente invention.

De façon générale, l'invention porte sur une composition thermo et photo stable comprenant un hydrogel formé d'un complexé de xanthane et chitosane, l'hydrogel comprenant au moins une substance thermo ou photo sensible choisie parmi les substances suivantes : vitamines, acides aminés, acides nucléiques et tripeptides, l'hydrogel étant adapté à relarguer ces dites substances thermo ou photo sensibles chez un sujet animal ou humain. Préférablement, les substances thermo ou photo sensibles seront présentes dans une proportion de 5 à 25 % du poids total de la composition. Plus préférablement, ces substances seront choisies parmi les vitamines A, B, C, D, E et K.

L'invention porte aussi sur une méthode de fabrication de la composition inventive, la méthode comprenant les étapes suivantes :

(a) la dissolution des substances liposolubles dans de l'éthanol ;

(b) l'ajout de cette solution, sous agitation, à une solution de xanthane ;

(c) la pulvérisation du mélange de l'étape (b) à une solution de chitosane ;

(d) la récupération de l'hydrogel ainsi formé ;

toutes les étapes se faisant en l'absence d'oxygène et de lumière.

Dans une autre méthode selon l'invention, la composition de l'invention pourra être obtenue par les étapes suivantes :

(a) la pulvérisation d'une solution de xanthane dans une solution de chitosane ;
(b) la récupération de l'hydrogel ainsi formé sera caractérisée par une lyophilisation de l'hydrogel ;
(c) l'introduction de l'hydrogel lyophilisé dans une solution aqueuse comprenant les substances hydrosolubles pouvant être avantageusement stabilisées par l'ajout d'acides aminiques tels les L-cystéine, L-cystine et L-méthionine ou mélange de ces derniers ou par l'ajout de tripeptides ;
(d) incorporation des substances hydrosolubles dans l'hydrogel par diffusion des substances hydrosolubles lors du gonflement de l'hydrogel, ledit hydrogel ayant un degré de gonflage de 2000 % ou plus.

Sous un aspect additionnel, l'invention présente une méthode d'utilisation de ces hydrogels en dermatologie ou comme complément alimentaire.

Figure 1 Complexation entre le chitosane et xanthane
Figure 2 Schéma du montage pour l'étude cinétique
Figure 3 Masse de vitamine C libérée en fonction du temps
Echantillon codé VS2L. Hydrogel CHITOXAN$^{MC}$-Vit. C préparé à partir de CHITOXAN$^{MC}$ avec degré de gonflage ($\alpha$) = 1800 %
Figure 4 Vitesse de libération de la vitamine C en fonction du temps.
Echantillon codé VS2L
Figure 5 Masse de vitamine C en fonction du temps
Echantillon codé VS2R. Hydrogel CHITOXAN$^{MC}$-Vit. C préparé à partir de CHITOXAN$^{MC}$ avec degré de gonflage ($\alpha$) = 2200 %
Figure 6 Vitesse de libération de la vitamine C en fonction du temps
Echantillon codé VS2R
Figure 7 Variation du pourcentage de la vitamine C libérée en fonction du temps
Echantillon VS2M Hydrogel CHITOXAN$^{MC}$-Vit. C préparé à partir de CHITOXAN$^{MC}$ avec degré de gonflage ($\alpha$) = 3500 %

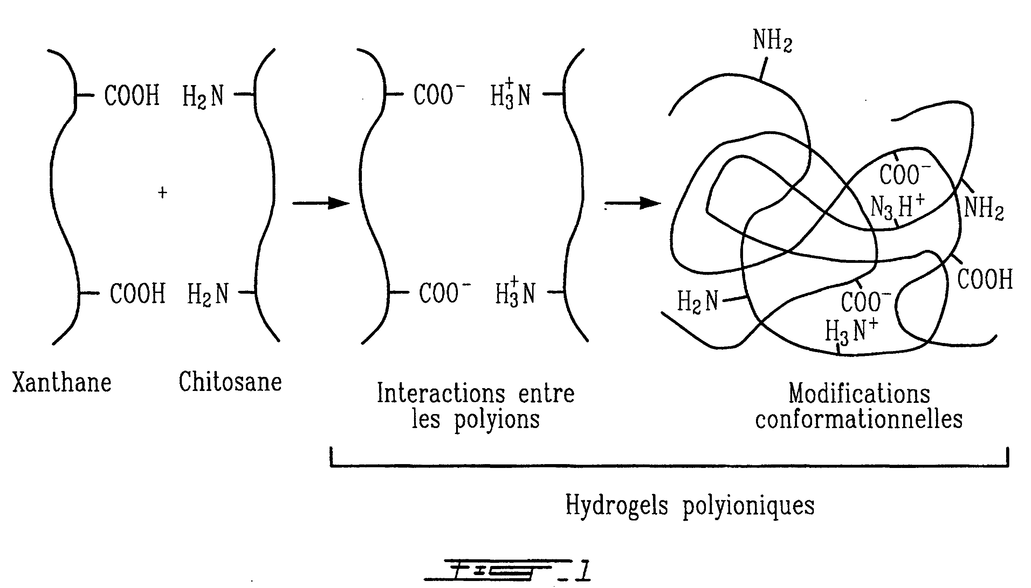

La présente invention détaillée révèle des compositions alimentaires et dermatologiques comprenant un ou plusieurs ingrédients actifs photo ou thermo sensibles à la dégradation. Ces compositions comprenant un hydrogel polyionique formé d'un complexe de chitosane et xanthane pouvant contenir et protéger les ingrédients actifs photo et thermo sensibles ainsi que bonifier leur activité par un effet de libération contrôlée.
Il est aussi décrit deux méthodes d'incorporation des ingrédients actifs dans l'hydrogel de chitosane et xanthane. Les ingrédients actifs liposolubles sont introduits dans l'hydrogel par une première méthode lors de la fabrication de l'hydrogel. Les ingrédients actifs hydrosolubles sont introduits par une méthode de diffusion dans l'hydrogel faisant suite à la fabrication de l'hydrogel.
La présente invention démontre, de façon surprenante et innovatrice, la possibilité d'introduire diverses vitamines ou autres ingrédients tels que acides aminés, acides nucléiques et tripeptides dans un hydrogel pour ensuite relarguer ces substances par différentes voies, tels que doses orales, suppositoires, crèmes, onguents, gels, solutions, dispositifs transcutanés (« patch »). De surcroît, la présente invention révèle la possibilité d'introduire et de solubiliser dans un même hydrogel des vitamines liposolubles ou hydrosolubles.
Finalement, il est aussi décrit une méthode de fabrication d'un complément alimentaire et d'une crème dermatologique incorporant l'hydrogel de la présente invention.
Il est à noter que les termes "dermatologie" et "dermatologique" sont employés dans leur sens large incluant ainsi toutes applications cosmétologique et cosmétique. De plus, ces termes s'étendent à des applications sur la peau ou sur les phanères.
Le terme "complément alimentaire" s'entend aussi dans son sens large incluant ainsi toute préparation alimentaire, le complément étant utilisé dans un rôle nutritionnel ou thérapeutique ou encore dans un simple rôle physique dans la préparation alimentaire, par exemple, en tant qu'agent texturant, de remplissage ou de contrôle de viscosité.
Les compositions de la présente invention sont destinées principalement au humains mais peuvent aussi avoir application dans le domaine vétérinaire.
Se référant à la figure 1, il est constaté que l'hydrogel utilisé dans la présente invention est un complexe à base de chitosane et xanthane issu d'une réaction ionique entre ces deux polyions. Une méthode de fabrication de l'hydrogel est décrite dans le brevet américain 5,720,206 accordé au même cessionnaire que la présente demande. Tel qu'illustré dans la figure 1, le xanthane et le chitosane forment un complexe c'est-à-dire l'établissement de divers liens ioniques entre les molécules de chitosane et de xanthane, formant ainsi l'hydrogel.

**Inclusion de vitamines liposolubles dans l'hydrogel chitosane-xanthane**

<u>vitamine A</u>

La vitamine A (Retinol) est un produit très sensible à la lumière et à l'oxygène. L'utilisation de ce produit dans une crème à titre d'exemple, ne peut se faire que si le produit est préalablement stabilisé contre les effets négatifs des agents précités. La méthode de la présente invention consiste à stabiliser la vitamine A dans un hydrogel composé de xanthane et de chitosane préparé selon la méthode décrite dans le brevet américain 5,620,706.

Exemple 1 inclusion de la vitamine A durant la fabrication

Une solution (100ml) de vitamine A (10 - 20 % w/v) dans l'alcool éthylique a d'abord été préparée. Cette solution est ajoutée, sous forte agitation, à 500 ml d'une solution aqueuse de xanthane, 0,65 % w/v, pour obtenir une concentration finale en vitamine A de 1,66 - 3,33 % w/v. La solution peut être conservée à 3°C. Par la suite, un système de pulvérisation. est utilisé pour ajouter la solution de vitamine A - xanthane à 800 ml d'une solution aqueuse de chitosane 0,65 % w/v. La réaction doit se poursuivre pendant 30 min. Le gel formé doit être filtré et lavé avec de l'eau pour atteindre un pH de 6.8. Pour augmenter la stabilité finale du gel, un dernier lavage avec une solution de bicarbonate de sodium 1 % w /v amène le gel à un pH de 7.5. Le gel est par la suite congelé et lyophilisé. Toutes les opérations, Incluant la congélation, se font en l'absence d'oxygène et de lumière.

Exemple 2 inclusion de la vitamine A par diffusion

En utilisant un hydrogel à base de xanthane-chitosane possédant un degré de gonflage ($\alpha$) d'au minimum 2000 %, il est possible d'introduire la vitamine A par diffusion. Le degré de gonflage se définit selon la formule suivante :

$$\alpha = 100\ \%\ \mathrm{X}\ \frac{\text{masse d'hydrogel gonflé à l'équilibre - masse d'hydrogel sec}}{\text{masse d'hydrogel sec}}$$

Dans ces conditions, on diminue de façon importante le temps de manipulation prévenant ainsi la dégradation de la molécule. Pour ce faire, il suffit de dissoudre 0,07 g de vitamine A dans 1 ml d'alcool éthylique (96%) et d'y ajouter 1,5 g du complexe xanthane-chitosane lyophilisé avec $\alpha$ = 2500 %.

Une légère agitation permet d'obtenir une pâte homogène. On ajoute alors 2ml d'alcool éthylique et 200µl d'eau toujours sous légère agitation et on place le tout à 4°C pendant 24 h à l'abri de la lumière. L'alcool est par la suite évaporé à 4°C. Le produit fini possède une concentration de 46 mg de vitamine A/g produit lyophilisé.

Exemple 3 inclusion de la vitamine E

Pour l'incorporation de la vitamine E, la méthode (1) développée pour la vitamine A est appliquée. La concentration en vitamine E peut atteindre jusqu'à 20 %.

Exemple 4 inclusion de la vitamine K

En ce qui concerne la vitamine K, la même méthode (1) que pour la vitamine A est utilisé avec une concentration en vitamine K pouvant atteindre 20 %.

**Inclusion de vitamines solubles dans l'eau**

Pour les produits bioactifs solubles dans l'eau il est préférable d'utiliser la méthode de diffusion dans l'hydrogel lyophilisé pour éviter les pertes inévitables de produits pendant la réaction entre le xanthane et le chitosane. Il est nécessaire dans ces conditions d'avoir un hydrogel avec un degré de gonflage d'au minimum 2000 %.

Exempte 5 Inclusion de la vitamine C

Parce que la vitamine C présente une forte action oxydo-réductrice par rapport au chitosane, une nouvelle méthode d'inclusion a été développée. Cette méthode comprend deux (2) étapes :

Etape 1 La préparation du complexe xanthane-chitosane (CHITOXAN$^{MC}$), c'est-à-dire l'hydrogel polyionique ;
Etape 2 L'incorporation de la vitamine C.

1. Préparation du complexe xanthane-chitosane

Le complexe CHITOXAN$^{MC}$ se prépare par la méthode décrite antérieurement, soit celle du brevet américain 5,620,706. Le chitosane utilisé pour préparer ce complexe a typiquement un poids moléculaire compris entre 250,000 et 350,000, et l'hydrogel un $\alpha \geq 2000$ %.
Le CHITOXAN$^{MC}$ est moulu pour obtenir une fine poudre avec des particules ayant un diamètre compris entre 250 et 500µm.

2. Incorporation de la vitamine C

2a. stabilisation par acides aminiques

A 10 ml d'eau on ajoute 1g de vitamine C, 0,06g de L-cystéine, 0,02g de L-cystine, 0,02g de L-méthionine. Par la suite, on ajoute 1g du CHITOXAN$^{MC}$ lyophilisé avec des particules de 250 - 500 µm. Le mélange ne doit pas présenter de phase liquide en excès. Si nécessaire, de l'eau peut être ajoutée. On maintient le mélange pendant 2 heures pour arriver à l'équilibre d'hydratation. Toutes les opérations doivent se faire en l'absence de lumière. Le mélange est par la suite congelé et lyophilisé. La transformation en poudre du produit final donne des particules variant entre 50 et 125µm.
La vitamine C ainsi incorporée et hydratée présente une stabilité sans aucun changement de coloration, de 2 semaines à 45°C et de 20 semaines pour le gel sec à cette même température. Il est possible d'utiliser comme agent de stabilisation, de l'acide tartrique 0,1 % de l'acide métaphosphorique 0,03 % ou de l'acide citrique 0,1 %. Le pourcentage pondéral est défini par rapport au complexe CHITOXAN$^{MC.}$

2b. Stabilisation par tripeptides

On utilise la méthode 2a, en remplaçant les acides aminés par le tripeptide avec acides aminiques sulfurés. A 10 ml d'eau, on ajoute 1g de vitamine C et 0,002 g de gluthatione. Après 5 minutes d'agitation, on introduit 1 g CHITOXAN$^{MC}$ et on agite légèrement jusqu'à l'obtention d'une pâte homogène. On laisse en repos pendant 2 heures pour arriver à l'équilibre d'hydratation. La pâte est congelée et lyophilisée. Toutes les opérations doivent se faire en l'absence de lumière.

**Extraction et dosage de ta vitamine C incluse dans le CHITOXAN$^{MC}$**

Extraction

Solvant d'extraction : solution aqueuse d'acide métaphosphorique 3 % (w/v), 8 % acide acétique (v/v)

Méthode : Dans un tube à centrifuger de 50 ml protégé contre la lumière, on introduit de 20 jusqu'à 30 mg de CHITOXAN$^{MC}$ + Vit. C lyophilisé et 40 ml du solvant d'extraction. Agiter pendant 60 minutes avec un barreau magnétique. Finalement, la suspension est centrifugée (4000 rpm) et le surnageant est analysé.

Détermination quantitative de la vitamine C

Avant de procéder à la détermination quantitative de la vitamine C, il faut mesurer le maximum d'absorption dans un spectrophotomètre UV-VIS. Le standard se prépare en utilisant une solution de vitamine C, qui a servi à la fabrication du CHITOXAN$^{MC}$-Vit. C, dans le solvant d'extraction. Notre test a donné un maximum d'absorption à 243 nm pour la vitamine C type ALDRICH, pureté 98 %.
Après avoir évalué le maximum d'absorption, on détermine la courbe étalon (absorption en fonction de la concentration de la vitamine C). Dans une fiole jaugée, on prépare une solution de vitamine C dans le solvant d'extraction à une concentration de 1,3 mg / ml. La solution est préparée juste avant de procéder à l'analyse. Par dilution successive, on mesure la dépendance absorption / concentration (mg / ml).
La concentration du surnageant obtenu dans l'extraction est déterminée par photocolorimétrie à 243 nm et calculée à partir de la courbe étalon.
La concentration de la vitamine C dans l'échantillon préparé par la méthode 2a est de 49.6%.

**Inclusion du CHITOXAN$^{MC}$-Vit. C dans une base de crème**

1 g de Chitoxan$^{MC}$-Vit. C est hydraté avec de l'eau jusqu'à l'obtention d'une pâte crémeuse. Par la suite, il suffit de peser la pâte et de calculer la concentration donnée en vitamine C. Subséquemment, la pâte est introduite sous forte agitation dans la base de crème pour obtenir une concentration finale désirée en vitamine, préférablement de 5 à 25% poids.

**Détermination de la stabilité de la vitamine C incluse dans une base de crème**

10 g de la préparation obtenue par inclusion de CHITOXAN$^{MC}$-Vit. C dans une base crème est introduite dans un tube protégé de la lumière. Ce tube est par la suite chauffé à 45°C. Pour déterminer le taux de dégradation de la vitamine C dans la base de crème, on prend un échantillon qui correspond à environ 10 - 30 mg de vitamine C. Cet échantillon est prélevé chaque jour et ce pendant les 4 premiers jours et par la suite tous les 2 jours pendant 30 jours. Si les échantillons ne sont pas analysés tout de suite, ils doivent être conservés au congélateur (-4°C).

Les échantillons prélevés sont analysés de la manière décrite précédemment (toujours en utilisant le solvant d'extraction).

A partir des échantillons VS2L, VS2R, et VS2M, on a préparé des crèmes avec une concentration de 5% à 25% poids en vitamine C. En utilisant la méthode décrite, on a trouvé une bonne stabilité de la vitamine C incluse dans le CHITOXAN$^{MC}$; aucune coloration de la crème n'a été observée après un chauffage à 45°C pendant 30 jours alors que la décomposition de la vitamine C a été de 15%. Pour une crème préparée à partir de vitamine C libre, on a trouvé, dans les mêmes conditions, une décomposition de 98% et une coloration orangée.

**Cinétique de libération**

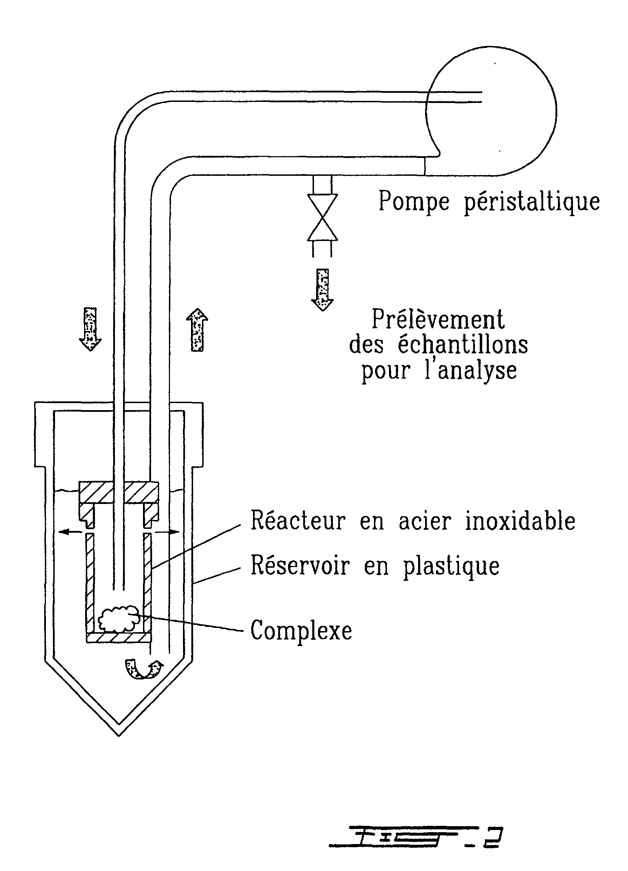

Se référant au tableau 1 ci-dessous, trois types d'hydrogels CHITOXAN$^{MC}$-Vit. C furent étudiés. Tel qu'illustré par la figure 2, la cinétique de relargage de la vitamine C est déterminée en introduisant une quantité précise du CHITOXAN$^{MC}$-Vit. C dans le réacteur.

Tableau 1

| Code des préparations CHITOXAN$^{MC}$-Vit. C | Degré de gonflage ($\alpha$) du CHITOXAN$^{MC}$ (%) | Concentration de vitamine C dans le CHITOXAN$^{MC}$-Vit. C (%) |
|---|---|---|
| VS2L | 1800 | 50,3 |
| VS2R | 2200 | 49,6 |
| VS2M | 3500 | 51,0 |

Le solvant (mélange 3 % w/v acide métaphosphorique et 8 % w/v acide acétique) pénètre par le tuyau central directement dans le réacteur où il entre en contact avec le CHITOXAN$^{MC}$-Vitamine C. La vitamine C se libère graduellement du complexe et entre en solution dans le solvant. Le solvant s'échappe du réacteur par les petits orifices et se retrouve dans le tube extérieur. Avec cette méthode, on assure une circulation constante de solvant autour de l'échantillon à analyser.

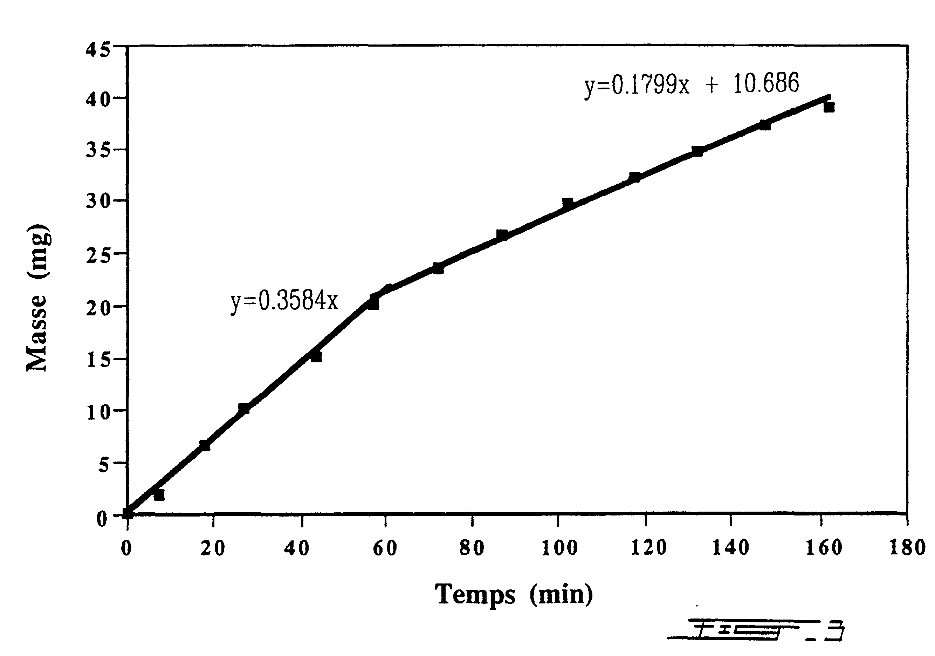

Se référant maintenant à la figure 3, il est vu que la masse de vitamine C libérée montre une relation linéaire en fonction du temps (cinétique d'ordre zéro) avec une cassure nette après 60 minutes dans le cas du CHITOXAN$^{MC}$-Vit. C de type VS2L et 40 minutes dans la cas du CHITOXAN$^{MC}$-Vitamine C de type VS2R.

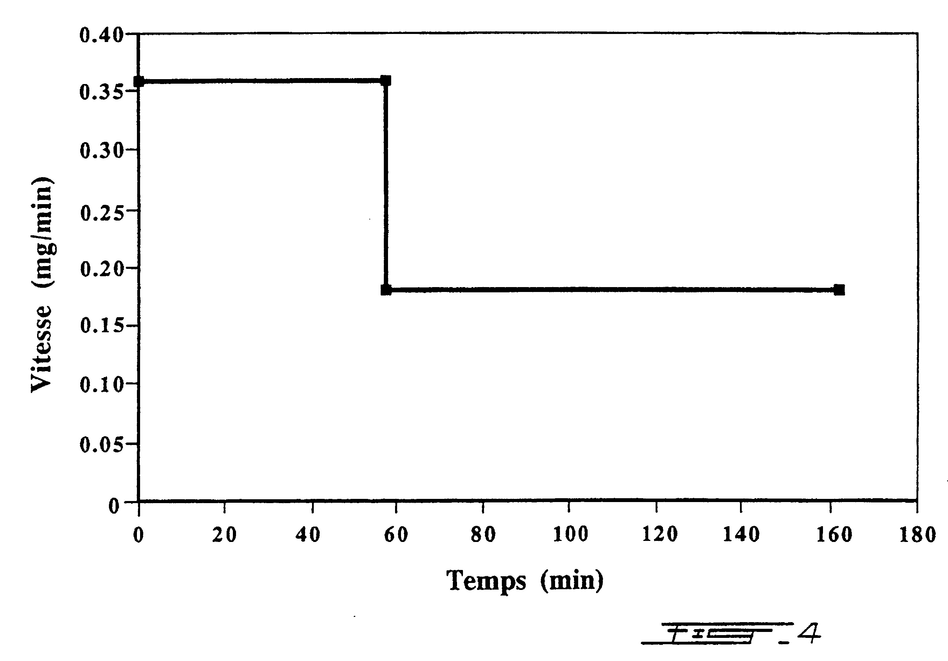

Se référant maintenant à la figure 4, il est vu dans un premier cas (CHITOXAN$^{MC}$-Vit. C de type VS2L) que la vitamine C diffuse à une vitesse constante de 0.36 mg/min pendant la première période, puis la vitesse diminue de moitié pour les 100 minutes suivantes.

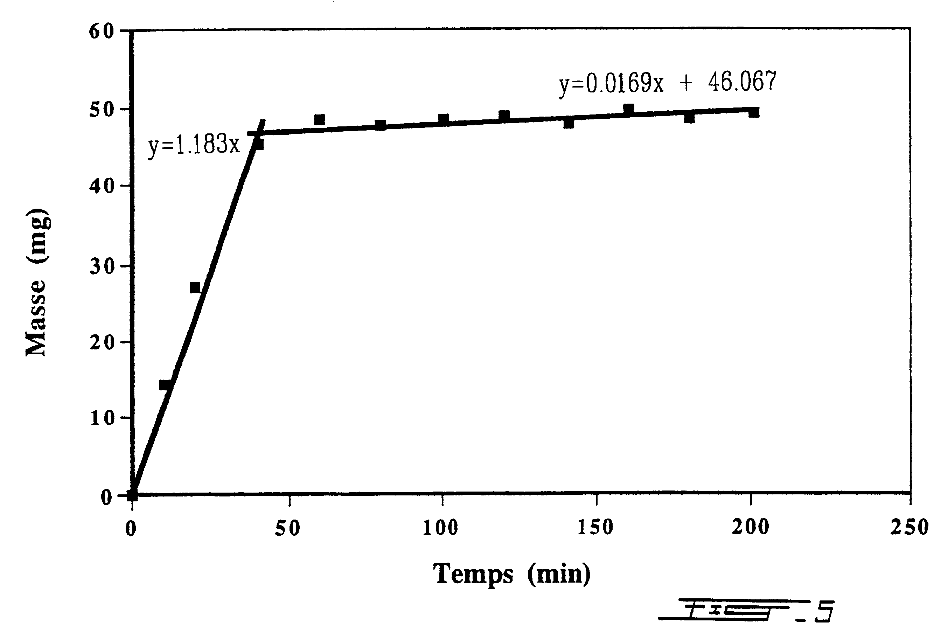

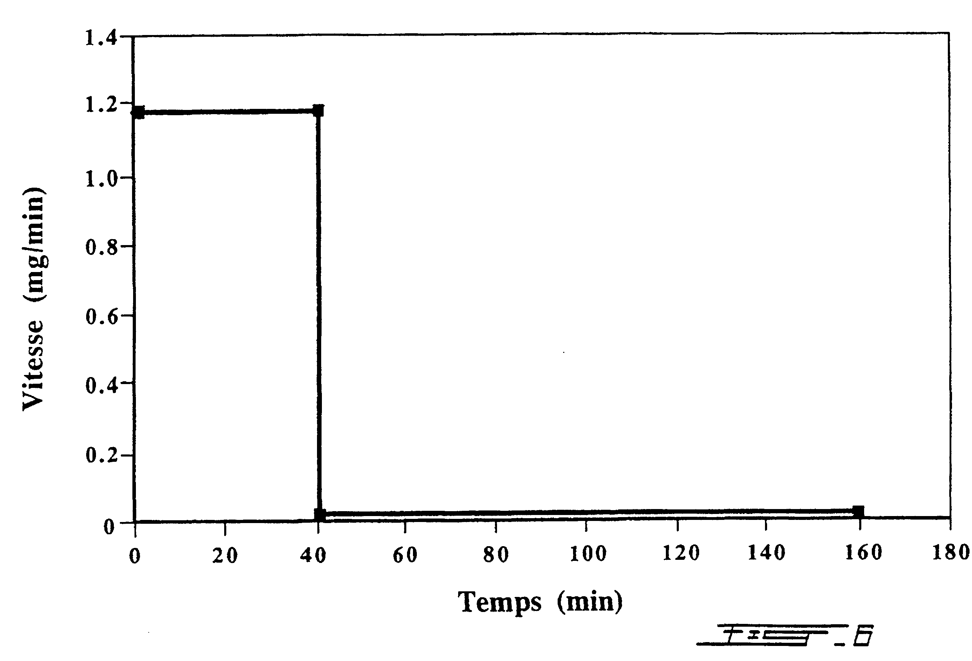

Dans le deuxième cas illustré aux figures 5 et 6 la diffusion s'effectue beaucoup plus rapidement dans les 40 premières minutes avec une vitesse de 1.18 mg/min, puis elle est presque nulle avec une valeur de 0.02 mg/min. La figure 5 montre la masse de vitamine C en fonction du temps pour l'échantillon codé VS2R. Hydrogel CHTOXAN$^{MC}$-Vit. C préparé à partir de CHITOXAN$^{MC}$ avec un degré de gonflage ($\alpha$) = 2200 %. La figure 6 montre la vitesse de libération de la vitamine C en fonction du temps pour l'échantillon codé VS2R.

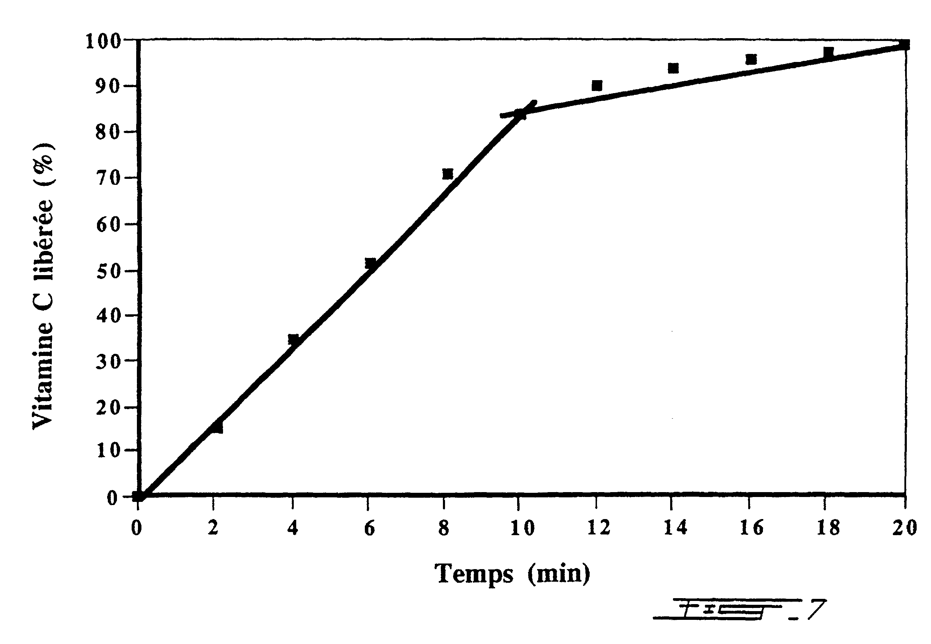

La figure 7 montre la variation du pourcentage de la vitamine C libérée en fonction du temps pour un échantillon VS2M Hydrogel CHITOXAN$^{MC}$-Vit.C préparé à partir de CHITOXAN$^{MC}$ avec un degré de gonflage ($\alpha$) = 3500 %. Pour cet échantillon, une libération de 85 % de la vitamine C dans les premiers 10 minutes d'élution fut constatée.

**Inclusion du CHITOXAN$^{MC}$-Vit. C dans une préparation alimentaire**

L'Hydrogel CHITOXAN$^{MC}$-Vit. C et d'autres hydrogels chitosane-xanthane contenant vitamines, acides nucléiques, acides aminés et oligopeptides peuvent aussi être utilisés dans des préparations alimentaires hydratées telles que gelées, sauces et sirops ainsi que dans des préparations déshydratées.

Les caractéristiques physiques de l'hydrogel chitosane-xanthane choisi détermineront la structure et la texture plus ou moins visqueuse de l'hydrogel. Ce choix d'hydrogel selon la présente invention permettra donc d'adapter l'hydrogal à diverses applications alimentaires.

De surcroît, la présente invention comporte la mise en pastille de la poudre CHITOXAN$^{MC}$-Vit. C avec d'autres ingrédients actifs, si requis. Il est aussi prévu la mise en pastille d'une poudre lyophilisée d'autres hydrogels chitosane-xanthane contenant vitamines diverses, acides aminés, acides nucléiques, tripeptides ou une combinaison parmi ces ingrédients actifs.

Il va de soi que la présente demande peut recevoir aménagements et variantes sans pour autant sortir du cadre de la présente invention telle que décrite.

## Revendications

**1.** Composition thermo et photo stable comprenant un hydrogel formé d'un complexe de xanthane et chitosane, ledit hydrogel comprenant au moins une substance thermo ou photo sensible choisie parmi les substances suivantes: vitamines, acides aminés, acides nucléiques et tripeptides pouvant être relarguées sur plusieurs heures chez un sujet animal ou humain.

**2.** Composition selon la revendication 1 dans laquelle les substances thermo ou photo sensibles sont des vitamines dans une proportion de 5 à 25 % du poids total de la composition.

**3.** Composition selon la revendication 2 dans laquelle les vitamines sont choisies parmi les vitamines A, B, C, D, E et K.

**4.** Composition selon la revendication 3 dans laquelle la vitamine est la vitamine A.

**5.** Composition selon la revendication 3 dans laquelle la vitamine est la vitamine C.

**6.** Composition selon la revendication 1 dans laquelle les substances thermo et photo sensibles sont aussi liposolubles.

**7.** Composition selon la revendication 1 dans laquelle les substances thermo et photo sensibles sont aussi hydrosolubles.

**8.** Méthode de fabrication de la composition de la revendication 6 comprenant les étapes suivantes :

(a) la dissolution des substances liposolubles dans l'alcool éthylique ;
(b) l'ajout de cette solution, sous agitation, à une solution aqueuse de xanthane ;
(c) la pulvérisation du mélange de l'étape (b) à une solution aqueuse de chitosane ;
(d) la récupération de l'hydrogel ainsi formé ;

toutes les étapes se faisant en l'absence d'oxygène et de lumière.

**9.** Méthode de fabrication de la composition de la revendication 7 comprenant les étapes suivantes :

(a) la pulvérisation d'une solution de xanthane dans une solution de chitosane ;
(b) la récupération de l'hydrogel ainsi formé, cette récupération étant **caractérisée par** une lyophilisation de l'hydrogel ;
(c) l'introduction de l'hydrogel lyophilisé dans une solution aqueuse comprenant les substances hydrosolubles ;
(d) incorporation des substances hydrosolubles dans l'hydrogel par diffusion des substances hydrosolubles lors du gonflement de l'hydrogel, ledit hydrogel ayant un degré de gonflage de 2000 % ou plus.

**10.** La méthode de fabrication de la revendication 9 dans laquelle, lors de l'étape (c), la stabilisation des substances hydrosolubles est favorisée par l'ajout d'acides aminiques.

**11.** La méthode de la revendication 10 dans laquelle les acides aminiques sont choisis parmi les L-cystéine, L-cystine et L-méthionine ou mélange de ces derniers.

**12.** La méthode de fabrication de la revendication 9 dans laquelle, lors de l'étape (c), la stabilisation des substances hydrosolubles est favorisée par l'ajout de tripeptides.

13. L'utilisation de la composition de l'une ou l'autre des revendications 1 à 7 dans la fabrication d'un produit dermatologique.

14. L'utilisation de la revendication 13 dans laquelle le produit dermatologique est une crème.

15. L'utilisation de la revendication 14 dans laquelle la substance thermo et photo sensible est la vitamine A.

16. L'utilisation de la composition de l'une ou l'autre des revendications 1 à 7 dans la fabrication d'un complément alimentaire.

**Patentansprüche**

1. Thermo- und photostabile Zusammensetzung, umfassend ein Hydrogel, gebildet aus einem Komplex von Xanthan und Chitosan, wobei besagtes Hydrogel mindestens eine thermo- oder photo-empfindliche Substanz umfaßt, ausgewählt aus den folgenden Substanzen: Vitamine, Aminosäuren, Nukleinsäuren und Tripeptide, die über mehrere Stunden bei einem Tier oder Mensch freigesetzt werden können.

2. Zusammensetzung nach Anspruch 1, bei der die thermo- oder photo-empfindlichen Substanzen Vitamine in einem Anteil von insgesamt 5 bis 25 Gew.% der Zusammensetzung sind.

3. Zusammensetzung nach Anspruch 2, bei der die Vitamine aus den Vitaminen A, B, C, D, E und K ausgewählt sind.

4. Zusammensetzung nach Anspruch 3, bei der das Vitamin Vitamin A ist.

5. Zusammensetzung nach Anspruch 3, bei der das Vitamin Vitamin C ist.

6. Zusammensetzung nach Anspruch 1, bei der die thermo- und photo-empfindlichen Substanzen auch fettlöslich sind.

7. Zusammensetzung nach Anspruch 1, bei der die thermo- und photo-empfindlichen Substanzen auch wasserlöslich sind.

8. Verfahren zur Herstellung einer Zusammensetzung nach Anspruch 6, umfassend die folgenden Schritte:

(a) Auflösung der fettlöslichen Substanzen in Ethylalkohol;
(b) Zugabe dieser Lösung, unter Rühren, zu einer wäßrigen Lösung von Xanthan;
(c) Sprühen der Mischung aus Schritt (b) in eine wäßrige Lösung von Chitosan;
(d) Erhalt des so gebildeten Hydrogels;

wobei alle Schritte in der Abwesenheit von Sauerstoff und von Licht stattfinden.

9. Verfahren zur Herstellung der Zusammensetzung nach Anspruch 7, umfassend die folgenden Schritte:

(a) Sprühen einer Lösung von Xanthan in eine Lösung von Chitosan;
(b) Erhalt des so gebildeten Hydrogels, wobei dieser Erhalt durch eine Lyophilisierung des Hydrogels charakterisiert ist;
(c) Einführung des lyophilisierten Hydrogels in eine wäßrige Lösung, umfassend die wasserlöslichen Substanzen;
(d) Einbau der wasserlöslichen Substanzen in das Hydrogel mittels Diffusion der wasserlöslichen Substanzen beim Aufquellen des Hydrogels, wobei besagtes Hydrogel einen Quellungsgrad von 2000% oder mehr hat.

10. Verfahren zur Herstellung nach Anspruch 9, bei dem während Schritt (c) die Stabilisierung der wasserlöslichen Substanzen durch die Zugabe von Aminosäuren begünstigt wird.

11. Verfahren nach Anspruch 10, bei dem die Aminosäuren ausgewählt sind aus L-Cystein, L-Cystin und L-Methionin oder eine Mischung der vorhergehenden.

**12.** Verfahren zur Herstellung nach Anspruch 9, bei dem während Schritt (c) die Stabilisierung der wasserlöslichen Substanzen durch die Zugabe von Tripeptiden begünstigt wird.

**13.** Verwendung der Zusammensetzung nach einem der Ansprüche 1 - 7 bei der Herstellung eines dermatologischen Produkts.

**14.** Verwendung nach Anspruch 13, bei der das dermatologische Produkt eine Creme ist

**15.** Verwendung nach Anspruch 14, bei der die thermo- und photo-empfindliche Substanz Vitamin A ist.

**16.** Verwendung der Zusammensetzung nach einem der Ansprüche 1 - 7 bei der Herstellung eines Nahrungsmittelzusatzes.

## Claims

**1.** Heat and light stable composition comprising a hydrogel formed of a complex of xanthan and chitosan, the said hydrogel including at least one heat or light sensitive substance selected from the following substances: vitamins, amino acids, nucleic acids and tripeptides able to be released over several hours in an animal or human subject.

**2.** Composition as described in claim 1, in which the heat or light sensitive substances are vitamins in a proportion of 5 to 25% of the total weight of the composition.

**3.** Composition as described in claim 2, in which the vitamins are selected from vitamins A, B, C, D, E and K.

**4.** Composition as described in claim 3, in which the vitamin is vitamin A.

**5.** Composition as described in claim 3, in which the vitamin is vitamin C.

**6.** Composition as described in claim 1, in which the heat and light sensitive substances are also fat-soluble.

**7.** Composition as described in claim 1, in which the heat and light sensitive substances are also hydrosoluble.

**8.** Method for manufacture of the composition of claim 6, comprising the following steps:

(a) the dissolving of the fat-soluble substances in ethyl alcohol;
(b) the addition of this solution, with agitation, to an aqueous xanthan solution;
(c) the spraying of the mixture of step (b) into an aqueous chitosan solution;
(d) the recovery of the hydrogel thus formed;

all the steps being performed in the absence of oxygen and light.

**9.** Method for manufacture of the composition of claim 7 comprising the following steps:

a) the spraying of a xanthan solution into a chitosan solution;
b) the recovery of the hydrogel thus formed, this recovery being ***characterised by*** freeze-drying of the hydrogel;
c) the introduction of the freeze-dried hydrogel into an aqueous solution including the hydrosoluble substances,
d) incorporation of the hydrosoluble substances in the hydrogel by diffusion of the hydrosoluble substances on expansion of the hydrogel, the said hydrogel having a degree of expansion of 2000% or more.

**10.** The manufacturing method of claim 9, in which, at step (c), stabilisation of the hydrosoluble substances is favoured by the addition of amino acids.

**11.** The method of claim 10, in which the amino acids are selected from L-cysteine, L-cystine and L-methionine, or a mixture of these.

**12.** The manufacturing method of claim 9, in which, at step (c), stabilisation of the hydrosoluble substances is favoured by the addition of tripeptides.

13. The use of the composition of one or the other of claims 1 to 7 in the manufacture of a dermatological product.

14. The use of claim 13 in which the dermatological product is a cream.

15. The use of claim 14 in which the heat and light sensitive substance is vitamin A.

16. The use of the composition of one or the other of claims 1 to 7 in the manufacture of a food complement.

Xanthane
Chitosane
COOH
$H_2N$
+
$COO^-$
$H_3^+N$
Interactions entre les polyions
$NH_2$
COOH
$N_3H^+$
$H_3N^+$
$H_2N$
Modifications conformationnelles
Hydrogels polyioniques

FIG - 1

Pompe péristaltique

Prélèvement des échantillons pour l'analyse

Réacteur en acier inoxidable

Réservoir en plastique

Complexe

y=0.1799x + 10.686
y=0.3584x
Masse (mg)
45
40
35
30
25
20
15
10
5
0
Temps (min)
0
20
40
60
80
100
120
140
160
180

FIG. 3

0.40
0.35
0.30
0.25
0.20
0.15
0.10
0.05
0
Vitesse (mg/min)
0
20
40
60
80
100
120
140
160
180
Temps (min)

FIG - 4

y=0.0169x + 46.067
y=1.183x
Masse (mg)
Temps (min)
0
10
20
30
40
50
60
0
50
100
150
200
250


FIG - 5

Vitesse (mg/min)
1.4
1.2
1.0
0.8
0.6
0.4
0.2
0
0
20
40
60
80
100
120
140
160
180
Temps (min)
FIG - 6

Vitamine C libérée (%)
100
90
80
70
60
50
40
30
20
10
0
0
2
4
6
8
10
12
14
16
18
20
Temps (min)

FIG - 7